# EUROPEAN PATENT SPECIFICATION

(11) **EP 4 234 578 B1**
(45) Date of publication and mention of the grant of the patent: **12.11.2025**
(21) Application number: 21883129.5
(22) Date of filing: 14.10.2021
(51) Int. Cl.: C07K 14/47, C07K 7/06, C07K 16/28, C12N 15/62, A61K 39/39, A61K 31/704, A61P 35/00, C07K 19/00, A61K 9/00, A61K 9/51, A61K 39/00, B82Y 5/00, C07K 14/705

(54) **FERRITIN NANOCAGE FUSED WITH PD-L1-BINDING PEPTIDE 1 AND USE THEREOF AS ANTICANCER IMMUNOTHERAPY AGENT**
FERRITIN-NANOKÄFIG, FUSIONIERT MIT PD-L1-BINDENDEM PEPTID 1 UND VERWENDUNG DAVON ALS ANTIKREBSIMMUNTHERAPIEMITTEL
NANOCAGE DE FERRITINE FUSIONNÉE AVEC LE PEPTIDE 1 DE LIAISON AU PD-L1 ET SON UTILISATION EN TANT QU'AGENT D'IMMUNOTHÉRAPIE ANTICANCÉREUSE

(30) Priority: 23.10.2020 KR 20200138075
(43) Date of publication of application: 30.08.2023
(73) Proprietor: Kyungpook National University Industry-Academic Cooperation Foundation, Daegu 41566 (KR)
(72) Inventor: KIM, Soyoun, Daegu 42093 (KR); YOO, Jae Do, Daegu 41944 (KR); JEON, In Seon, Suncheon-si Jeollanam-do 57905 (KR); KIM, Min-Seong, Daegu 41237 (KR); LEE, Byung-Heon, Daegu 42038 (KR)
(74) Representative: Roos, Peter
(86) International application number: PCT/KR2021/014261
(87) International publication number: WO 2022/086058

(56) References cited:
- EP-A1- 3 501 509
- WO-A1-2018/053434
- WO-A1-2019/117690
- KR-B1- 101 888 675
- US-A1- 2007 065 427
- GURUNG SMRITI ET AL: "Phage display-identified PD-L1-binding peptides reinvigorate T-cell activity and inhibit tumor progression", BIOMATERIALS, ELSEVIER, AMSTERDAM, NL, vol. 247, 20 March 2020 (2020-03-20), XP086130673, ISSN: 0142-9612, [retrieved on 20200320], DOI: 10.1016/J.BIOMATERIALS.2020.119984
- TRUFFI MARTA; FIANDRA LUISA; SORRENTINO LUCA; MONIERI MATTEO; CORSI FABIO; MAZZUCCHELLI SERENA: "Ferritin nanocages: A biological platform for drug delivery, imaging and theranostics in cancer", PHARMACOLOGICAL RESEARCH, ELSEVIER, AMSTERDAM, NL, vol. 107, 9 March 2016 (2016-03-09), AMSTERDAM, NL, pages 57 - 65, XP029533463, ISSN: 1043-6618, DOI: 10.1016/j.phrs.2016.03.002
- FEDERICA PALOMBARINI, ELISA DI FABIO, ALBERTO BOFFI, ALBERTO MACONE, ALESSANDRA BONAMORE: "Ferritin Nanocages for Protein Delivery to Tumor Cells", MOLECULES, vol. 25, no. 4, pages 825, XP055708580, DOI: 10.3390/molecules25040825
- JEON IN SEON, YOO JAE DO, GURUNG SMRITI, KIM MINSEONG, LEE CHANJU, PARK EUN JUNG, PARK RANG-WOON, LEE BYUNGHEON, KIM SOYOUN: "Anticancer nanocage platforms for combined immunotherapy designed to harness immune checkpoints and deliver anticancer drugs", BIOMATERIALS, ELSEVIER, AMSTERDAM, NL, vol. 270, 1 March 2021 (2021-03-01), AMSTERDAM, NL , pages 120685, XP055924220, ISSN: 0142-9612, DOI: 10.1016/j.biomaterials.2021.120685

## Description

### Technical Field

The present disclosure relates to a ferritin nanocage fused with a PD-L1-binding peptide 1 and a use thereof as an anticancer immunotherapy agent, and to a use of a ferritin nanocage fused with a PD-L1-binding peptide 1 for immune checkpoint control and drug delivery complex anticancer immunotherapy.

### Background Art

Cage proteins are proteins capable of forming macromolecules tens to hundreds of times the molecular weight of monomers due to precise self-assembly properties of low molecular weight monomers. In nature, it includes viral capsid proteins, ferritins, heat shock proteins, and DPS proteins, and each monomers constituting a cage show very regular and precise interactions with neighboring monomers, while the inside of the cage is empty. Container-like properties of the cage protein as described above make the inside and the outside isolated to ensure high applicability in the medical field as a drug delivery.

In the field of cage protein-applied substance transport, studies on viral vectors and non-viral vectors are actively underway. To date, adenoviruses as the viral vectors have been studied, and ferritins and heat shock proteins as the non-viral vectors. However, in the case of conventional viral vectors, there have been raised issues concerning in vivo safety due to genes possessed by virus itself.

Ferritin protein, a type of intracellular protein, that functions to store and release iron. Ferritin is generally in the form of a hollow spherical cage in vivo and composed of 24 subunits that are divided into heavy chains and light chains depending on the structure.

Meanwhile, the development in cancer immunotherapy has reached an important point of inflection in the history of cancer treatment. Programmed cell death (PD-1), one of the immune checkpoint molecules, has become an attractive therapeutic target for several cancers. PD-1 is upregulated in T cells upon activation and abundant in exhausted T cells commonly seen in tumor-infiltrating lymphocytes. Blocking the interaction of PD-1 with its ligand, excellent antitumor response and clinical effects were observed in some patients. EP 3 501 509 A1 relates to a nanocage formed by self-assembly of a fusion protein including a phagocytosis enhancing protein and a self-assembling protein, and a protein nanocage complex in which an immunogenic cell death inducer loaded in the nanocage as an active ingredient. WO 2018/053434 A1 relates to a protein nanocage formulation with enhanced mucus penetration capability and colloidal stability that provides controlled delivery of therapeutic, prophylactic, or diagnostic agents to tumors. GURUNG, SMRITI et al: "Phage display-identified PD-L1-binding peptides reinvigorate T-cell activity and inhibit tumor progression", Biomaterials, Elsevier, Amsterdam, NL, vol. 247, 20 March 2020 is a scientific article about phage display-identified PD-L1-binding peptides.

In light of the above, it is necessary to develop a drug delivery platform that binds to PD-1 to increase the immune checkpoint inhibitory function and stability of peptides that block the interaction of PD-1 and PD-L1, and is capable of effectively transporting to cancer tissues.
Disclosure of the Invention The scope of the invention is defined in the appended set of claims.

### Technical Goals

An object of the present disclosure is to provide a pharmaceutical composition for use in preventing or treating cancer comprising a ferritin nanocage comprising a fusion polypeptide in which a PD-L1-binding peptide consisting of an amino acid sequence represented by SEQ ID NO: 2 is conjugated to a N-terminal of a human ferritin heavy chain fragment consisting of an amino acid sequence represented by SEQ ID NO: 1 as an active ingredient.

In addition, an object of the present disclosure is to provide a polynucleotide encoding the fusion polypeptide, an expression vector including the polynucleotide, and a transformant transformed with the expression vector (except for humans).

In addition, an object of the present disclosure is to provide a pharmaceutical composition for preventing or treating cancer including the ferritin nanocage and an anticancer agent as active ingredients.

In addition, an object of the present disclosure is to provide a composition for drug delivery, including the ferritin nanocage as an active ingredient.

### Technical Solutions

In order to solve the above problem, the present disclosure provides a pharmaceutical composition for use in preventing or treating cancer as defined in the claims.

Also described is a polynucleotide encoding the fusion polypeptide.

Further described is an expression vector including the polynucleotide.

Also described is a transformant transformed with the expression vector (except for humans).

Accordingly, the present disclosure provides a pharmaceutical composition for preventing or treating cancer including the ferritin nanocage as an active ingredient.

The pharmaceutical composition for preventing or treating cancer can include the ferritin nanocage and an anticancer agent as active ingredients.

A composition for drug delivery is further described, including the ferritin nanocage as an active ingredient.

### Advantageous Effects

The present invention relates to a pharmaceutical composition for use in preventing or treating cancer, comprising a ferritin nanocage comprising a fusion polypeptide in which a PD-L1-binding peptide consisting of an amino acid sequence represented by SEQ ID NO: 2 is conjugated to a N-terminal of a human ferritin heavy chain fragment consisting of an amino acid sequence represented by SEQ ID NO: 1 as an active ingredient. The ferritin nanocage is fused with a PD-L1-binding peptide 1. The nanocage was prepared by fusing a human ferritin monomer with a peptide 1 (PD-L1pep1: CLQKTPKQC) that binds to immune checkpoint receptor PD-L1 overexpressed in many cancers such as breast cancer, colorectal cancer, renal cancer, and glioblastoma, such that 24 Pd-L1prep1s are displayed when the cage is formed. The nanocage of the present disclosure exhibited an anticancer effect by effectively targeting colorectal cancer tissues, and when doxorubicin, an anticancer agent, is loaded in the nanocage, higher anticancer effects than PD-L1 antibodies in the same dose were shown. Accordingly, the nanocage is expected as a next-generation drug that surmounts the limitation of immune checkpoint blocking therapy using antibodies.

### Brief Description of Drawings

FIG. 1 shows results of designing and preparing fusion proteins (PpNF, PpLF, PpCF) in which PD-L1-binding peptide 1 (PD-L1pep1) is inserted into each of three junction points to identify differences in structure, biophysical properties, and binding force, wherein A) shows diagrams of gene sequences for PpNF, PpLF, and PpCF, B) shows results of verifying stable expression and purification of PpNF, PpLF, and PpCF in E. coli, and C) shows results of tertiary structural modeling of PpNF, PpLF, and PpCF.
FIG. 2 shows results of observing whether cages are formed and particle sizes of each prepared fusion protein by TEM and DLS, wherein A) shows results of transmission electron microscopy (TEM) analysis, and B) shows results of dynamic light scattering (DLS) analysis.
FIG. 3 shows results of comparative analysis of a binding force for each prepared fusion proteins, wherein A) shows results of culture cell binding assay, and B) shows results of protein binding assay by means of a surface plasmon resonance device.
FIG. 4 shows results of identifying an immune checkpoint inhibitory function of PpNF in vitro, wherein A) shows a diagram for observing an activity of PpNF following co-culture of cancer cells and immune cells, B) shows results of flow cytometer measurement to determine an immune checkpoint inhibitory effect of PpNF, and C) shows a result of measuring an amount of interferon gamma secreted by isolated T cells using an enzyme-linked immunosorbent assay to identify an immune checkpoint inhibitory effect of PpNF.
FIG. 5 shows results of identifying a tumor targeting ability of PpNF.
FIG. 6 shows results of identifying a glioblastoma targeting effect of an immune checkpoint blocking nanocage.
FIG. 7 shows results of analyzing anticancer and immune checkpoint inhibitory effects of PpNF in vivo.
FIG. 8 shows results of analyzing anticancer and immune checkpoint inhibitory effects in vivo in a combined therapy using PpNF and doxorubicin.
FIG. 9 shows results of determining biotoxicity of PpNF via blood and serologic analysis.

### Best Mode for Carrying Out the Invention

The present disclosure provides a fusion polypeptide in which a PD-L1-binding peptide having an amino acid sequence represented by SEQ ID NO: 2 is conjugated to a C-terminal or N-terminal of a human ferritin heavy chain fragment having an amino acid sequence represented by SEQ ID NO: 1.

Preferably, the fusion polypeptide may further include a linker between the human ferritin heavy chain fragment and the PD-L1-binding peptide. More preferably, the linker may have an amino acid sequence represented by SEQ ID NO: 3 (GG) or SEQ ID NO: 4 (GGGSGGGGSG), but is not limited thereto.

In addition, the present disclosure provides a ferritin nanocage including the fusion polypeptide. Specifically, the ferritin nanocage may be in a form in which the PD-L1-binding peptide is fused to an outer surface of ferritin.

As used herein, the term 'ferritin' is a type of intracellular proteins that function to store and release iron. Ferritin is generally in the form of a hollow spherical cage in vivo, and the cage is composed of 24 ferritin monomers which are divided into heavy chains and light chains depending on the structure. In the present disclosure, the ferritin protein may be used without limitation if each of the proteins has an activity to form a cage-like complex protein as a monomer.

In the present disclosure, human ferritin heavy chain fragments (1-161) having amino acid sequences represented by SEQ ID NO: 1 were used.

As used herein, the term 'PD-L1-binding peptide' refers to a peptide having an amino acid sequence (CLQKTPKQC) represented by SEQ ID NO: 2 and is indicated herein as "PD-L1-binding peptide 1" or "PD-L1pep1".

As used herein, the term "nanocage" refers to a cage that is formed by precise self-assembly properties of low molecular weight monomers and made of proteins having space inside. These include viral capsid proteins, ferritin, heat shock proteins, and DPS proteins. The nanocage of the present disclosure includes the fusion polypeptide of the present disclosure as a monomer constituting the nanocage. The term "self-assembly" as used herein refers to the property that certain molecules form a specific nanostructure by themselves without external special stimuli or artificial induction.

The nanocage of the present disclosure may be a complex protein in which the fusion polypeptides of the present disclosure are regularly arranged as monomers, and more preferably, the nanocage may be formed as the 24 fusion polypeptides of the present disclosure are regularly arranged in three dimensions.

In addition, also described is a polynucleotide encoding the fusion polypeptide.

The term "polynucleotide" as used herein refers to a polymer of deoxyribonucleotides or ribonucleotides that exist in a single-stranded or double-stranded form. The polynucleotide includes RNA genomic sequences, DNA (gDNA and cDNA), and RNA sequences transcribed therefrom and also includes analogs of natural polynucleotides unless otherwise specified.

The polynucleotide includes not only the nucleotide sequence encoding the fusion peptide, but also a sequence complementary to the sequence. The complementary sequence includes not only perfectly complementary sequences, but also substantially complementary sequences.

In addition, the polynucleotide may be modified. Such modifications include additions, deletions, or non-conservative substitutions or conservative substitutions of nucleotides. The polynucleotide encoding the amino acid sequence is construed to include a nucleotide sequence showing substantial identity to the nucleotide sequence. The substantial identity may refer to, when the nucleotide sequence and any other sequence are aligned to the maximum correspondence and the aligned sequence is analyzed using an algorithm commonly used in the art, a sequence showing at least 80% homology, at least 90% homology, or at least 95% homology.

In addition, further described is an expression vector including the polynucleotide.

In addition, also described is a transformant transformed with the expression vector (except for humans).

The term "vector" as used herein refers to a self-replicating DNA molecule used to carry a clonal gene (or another fragment of clonal DNA).

The term "vector" as used herein may refer to a plasmid, viral vector or other media known in the art capable of expressing the inserted nucleic acid in a host cell, and one that polynucleotides encoding the fusion polypeptide of the present disclosure are operably linked to a conventional expression vector known in the art. The expression vector may include an origin of replication to generally enable proliferation in a host cell, and one or more expression regulatory sequences (e.g., promoter, enhancer, etc.) to regulate expression, a selective marker, and a polynucleotide encoding the fusion polypeptide of the present disclosure operably linked to an expression regulatory sequence. The transformant may be transformed with the expression vector.

Preferably, the transformant may be obtained by introducing the expression vector including the polynucleotide encoding the fusion polypeptide of the present disclosure into a host cell by a method known in the art, for example, but not limited to, transient transfection, microinjection, transduction, cell fusion, calcium phosphate precipitation, liposome-mediated transfection, DEAE dextran-mediated transfection, polybrene-mediated transfection, electroporation, gene gun, and other known methods for introducing a nucleic acid into the cell (Wu et al., J. Bio. Chem., 267:963-967, 1992; Wu and Wu, J. Bio. Chem., 263:14621-14624, 1988).

Accordingly, the present disclosure provides a pharmaceutical composition for preventing or treating cancer including the ferritin nanocage as an active ingredient.

Preferably, the cancer may include breast cancer, colon cancer, rectal cancer, brain tumor, lung cancer, liver cancer, skin cancer, esophageal cancer, testicular cancer, kidney cancer, stomach cancer, bladder cancer, ovarian cancer, cholangiocarcinoma, gallbladder cancer, uterine cancer, cervical cancer, prostate cancer, head and neck cancer, pancreatic cancer, and squamous cell carcinoma, but is not limited thereto.

Preferably, the pharmaceutical composition may block binding of PD-1 and PD-L1 and inhibit immune checkpoints.

The present disclosure thus provides a pharmaceutical composition for preventing or treating cancer including the ferritin nanocage and an anticancer agent as active ingredients.

Preferably, the anticancer agent may be any one or more selected from the group consisting of doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, and nitrosourea, but is not limited thereto.

Preferably, the cancer may include breast cancer, colon cancer, rectal cancer, brain tumor, lung cancer, liver cancer, skin cancer, esophageal cancer, testicular cancer, kidney cancer, stomach cancer, bladder cancer, ovarian cancer, cholangiocarcinoma, gallbladder cancer, uterine cancer, cervical cancer, prostate cancer, head and neck cancer, pancreatic cancer, and squamous cell carcinoma, but is not limited thereto.

The pharmaceutical composition of the present disclosure may be prepared using a pharmaceutically suitable and physiologically acceptable adjuvant in addition to the active ingredient, and a solubilizing agent such as an excipient, a disintegrant, a sweetener, a binder, a coating agent, a swelling agent, a lubricant, a polishing agent, or a flavoring agent may be used as the adjuvant. The pharmaceutical composition of the present disclosure may be preferably formulated into a pharmaceutical composition by including one or more pharmaceutically acceptable carriers in addition to the active ingredient for administration. In the composition formulated as a liquid solution, pharmaceutically acceptable carriers may be sterile and biocompatible and used by mixing saline, sterile water, Ringer's solution, buffered saline, albumin injection, dextrose solution, maltodextrin solution, glycerol, ethanol and one or more of these components, while other conventional additives such as antioxidants, buffers, and bacteriostats may be added as needed. In addition, diluents, dispersants, surfactants, binders, and lubricants may be additionally added to formulate into an injectable formulation such as an aqueous solution, suspension, and emulsion as well as pills, capsules, granules, or tablets.

The pharmaceutical formulation of the pharmaceutical composition of the present disclosure may be granules, powder, coated tablets, tablets, capsules, suppositories, syrups, juices, suspensions, emulsions, drops or injectable solutions, and sustained-release preparations of the active compound. The pharmaceutical composition of the present disclosure may be administered in a conventional manner via intravenous, intraarterial, intraperitoneal, intramuscular, intraarterial, intraperitoneal, intrasternal, transdermal, intranasal, inhalational, topical, rectal, oral, intraocular or intradermal routes. The effective amount of the active ingredient of the pharmaceutical composition of the present disclosure refers to an amount required for preventing or treating a disease. Therefore, the effective amount may be controlled by various factors including the type of disease, the severity of the disease, the type and content of the active ingredient and other ingredients included in the composition, the type of formulation and the age, weight, general health status, sex and diet of a patient, administration time, administration route and secretion rate of the composition, treatment period, and drugs used in combination with. Although not limited thereto, for example, in the case of adults, when administered once to several times a day, the composition of the present disclosure may be administered at a dose of 0.1 ng/kg to 10 g/kg in the case of a compound when administered once to several times a day.

In addition, also described is a composition for drug delivery, including the ferritin nanocage as an active ingredient.

The ferritin nanocage according to the present disclosure may be used as an intelligent drug delivery that selectively delivers drugs to cancer tissues. If the previously known drug is loaded in the ferritin nanocage of the present disclosure to be used for cancer treatment, since the drug is selectively delivered only to cancer tissues and cancer cells by the ferritin nanocage of the present disclosure, it is possible to increase effectiveness of the drug while side effects of the drug on normal tissues may be significantly reduced.

The drug is an anticancer agent, and, as an anticancer agent that may be loaded in the ferritin nanocage of the present disclosure, there is no limitation in the use if it is for the conventional cancer treatment. For example, it may include doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, and nitrosourea.

### Modes for Carrying Out the Invention

Hereinafter, the present disclosure will be described in detail according to an example embodiment that does not limit the present disclosure. It is clear that the following example embodiments of the present disclosure are intended to embody the present disclosure and do not limit or restrict the scope of the present disclosure. Therefore, what may be easily inferred by an expert in the art to which the present disclosure pertains from the detailed description and example embodiments of the present disclosure is interpreted as falling within the scope of the present disclosure.

### <Example 1> Designing and purification of immune checkpoint (PpNF, PpLF, PpCF) proteins fused with PD-L1-binding peptide 1

### 1. Experimental Method

### (1) Preparation of PpNF, PpLF, and PpCF DNAs

A gene sequence of human ferritin heavy chain fragment (1-161) or that of human ferritin heavy chain (1-183) was obtained by amplifying by PCR in a cDNA library that was reverse-transcribed from human mRNA and inserted into pET-28a plasmids using a gene recombinant method. The gene sequence of PD-L1-binding peptide 1 was chemically synthesized and inserted into the nitrogen terminal of the human ferritin heavy chain fragment (1-161) using restriction enzymes KpnI and NheI or into the carbon terminal of the human ferritin heavy chain fragment (1-161) using restriction enzymes SpeI and XhoI. When inserting the peptide into the nitrogen terminal of ferritin, the peptide having GG sequence was inserted therebetween to increase the degree of freedom, and when inserting into the carbon terminal of ferritin, a longer flexible linker (GGGSGGGGSG) was inserted to increase the degree of freedom while preventing the peptide from being hidden inside the ferritin. In addition, the gene sequence of PD-L1-binding peptide 1 was inserted using the restriction enzymes BamHI and ApaI between genes corresponding to helix 4 and 5 of the human ferritin heavy chain (1-183) gene. A construct into which the peptide was inserted at the nitrogen terminal of ferritin was named PpNF, that inserted between helix 4 and 5 was named PpLF, and that inserted into the nitrogen terminal of ferritin was named PpCF.

### (2) Purification of PpNF, PpLF, and PpCF proteins

E. coli (BL21) transformed with each plasmid into which the sequences of PpNF, PpLF, and PpCF were inserted was inoculated into LB media and grown at 37°C in a shake incubator. When a value of OD₆₀₀ became 0.5, IPTG was added to the media to be 100 uM so as to induce protein overexpression. For PpNF and PpCF, lysis buffer was added into E. coli which was then disrupted by ultrasound. The lytic solution (supernatant) collected by centrifugation was mixed with Ni-NTA beads at 4°C for 1 hour. The lytic solution that did not bind to the bead was filtered out by a chromatographic column and the beads were washed with a wash buffer containing 30 mM imidazole. Endotoxins of E. coli were removed with a wash buffer containing 0.1% Triton X-114 which was then removed with a wash buffer. The protein was eluted and purified using elution buffers each containing 100 mM, 200 mM, 300 mM, and 500 mM imidazole. For PpLF, a lysis buffer was added to E. coli, disruption was performed by ultrasound, and then proteins were collected from the inclusion body collected by centrifugation. The proteins were fragmented into monomers with an extraction buffer containing 8 M urea and extracted from the inclusion body, and the isolated proteins (supernatant) were mixed with Ni-NTA beads at 4°C for 2 hours as above. Subsequently, for the wash buffer and elution buffer, 8 M urea was added to allow the protein to remain in a monomeric state. The lytic solution that did not bind to the beads was filtered out by a chromatographic column, and the beads were washed with the wash buffer containing 30 mM imidazole. Endotoxins of E. coli were removed with the wash buffer containing 0.1% Triton X-114 which was then removed by the wash buffer. The protein was eluted and purified using the elution buffer containing 300 mM imidazole. For the purified monomer protein, 8 M urea was slowly removed by dialysis to induce protein folding and formation of a ferritin nanocage again. For each protein of PpNF, PpLF, and PpCF, more than 95% homogeneous purified protein was collected by SDS PAGE.

### 2. Experimental Results

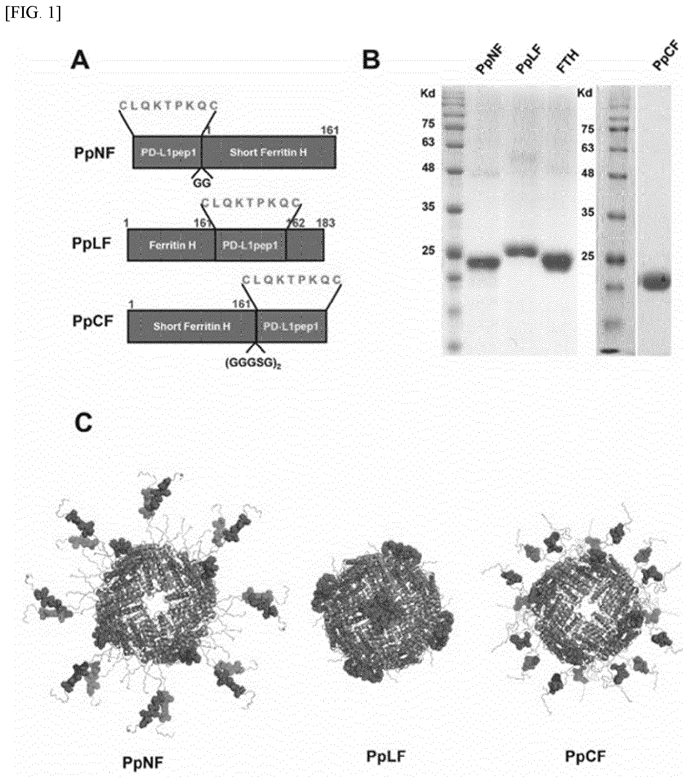

Human ferritin heavy chain fragments have three junction points, capable of peptide display on the surface when 24 monomers undergoes polymerization to form a cage, present on the N-terminal, a loop connecting the 4^{th} and 5^{th} helixes, and the C-terminal. Hereby, fusion proteins (PpNF, PpLF, PpCF) into which PD-L1-binding peptide 1 (PD-L1pep1) was inserted into each of these three junction points were designed and prepared to identify differences in structure, biophysical properties, and binding force (FIG. 1).

The diagrams of gene sequences for PpNF, PpLF, and PpCF are shown in FIG. 1A.

When electrophoresis of each protein of PpNF, PpLF, PpCF, and human ferritin heavy chain (FTH) was performed by SDS-polyacrylamide gel electrophoresis and then the protein bands were detected by staining the gel with Coomassie brilliant blue, stable expression and purification in E. coli were verified by detecting the protein band at the same position as the theoretical monomer size of each protein (FIG. 1B).

Through the tertiary structural models of PpNF, PpLF, and PpCF using MODELLAR v12 as a modeling program, it was found that the peptide was freely displayed on the surface in the case of PpNF, while the conformation of the peptide was implemented in the restricted range for PpLF. It was determined that PpCF had a degree of freedom higher than PpLF but not higher than that of PpNF (FIG. 1C).

### <Example 2> Observation whether the cage is formed and particle sizes of each prepared fusion protein by TEM and DLS

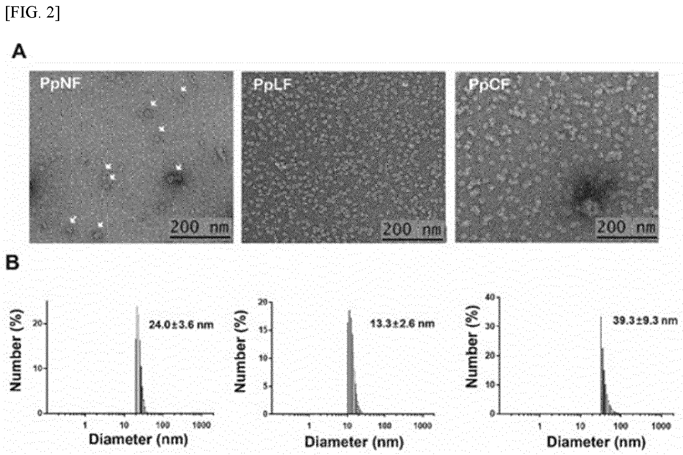

In E. coli in which PpNF, PpLF, and PpCF were overexpressed, each protein was purified using Ni-NTA beads, and transmission electron microscopy (TEM) analysis was performed to determine whether the proteins form ferritin nanocages (FIG. 2A). It was found that each protein formed the nanocage, and by performing dynamic light scattering (DLS) analysis, it was found that each cage size was about 24 nm for PpNF, about 13 nm for PpLF, and about 39 nm for PpCF (FIG. 2B).

### <Example 3> Comparative analysis on binding forces of each prepared fusion protein

### 1. Culture cell binding assay

### (1) Experimental Method

Confocal laser scanning microscopy (Confocal) was performed. After culturing triple negative breast cancer cells (MDA-MB 231) or colorectal cancer cells (CT26), a group treated with 25 ng/ml of interferon-γ and a group without treatment were cultured at 37°C for 24 hours. These cells are cancer cells expressing the PD-L1 receptor and are known to have expression increased upon interferon-γ treatment. MCF-7, a breast cancer cell that does not express PD-L1, was observed as a control. In order to block nonspecific binding of PD-L1 proteins, blocking was performed using 1% BSA dissolved in PBS at room temperature for 1 hour, then BSA was removed, and PpNF, PpLF, and PpCF (100 nM) were reacted at 4°C for 1 hour. After washing with PBS, a reaction was performed using anti-human ferritin heavy chain antibodies (1: 200 ratio) for 1 hour, followed by staining (green). After fixing with 4% paraformaldehyde, observation was performed under a confocal microscope. To observe the nucleus, DAPI was dissolved in PBS in a ratio of 1: 1000, and staining was performed at room temperature for 5-10 minutes (blue).

### (2) Experimental Results

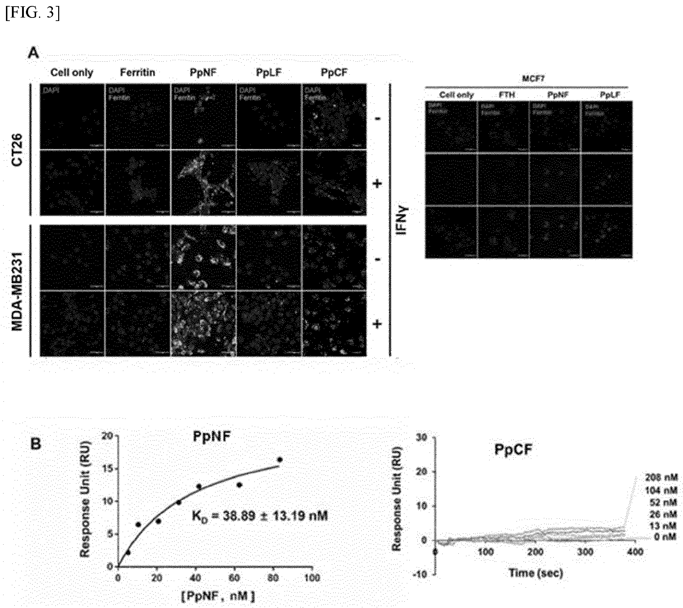

It was found that PpNF and PpCF fusion proteins bound in MDA-MB231 cells and CT26 cells expressing PD-L1. On the contrary, the binding did not take place in MCF-7 cells that do not express PD-L1, indicating that the binding was due to PD-L1. PpLF did not bind in MDA-MB231 cells and CT26 cells expressing PD-L1, despite the fact that the cage was well formed. Through the structural model, it was found that the peptide displayed on PpNF and PpCF was present on the surface in a conformation with a higher degree of freedom compared to PpLF, and a difference in the display pattern determined whether the binding takes place and the binding force (FIG. 3A).

### 2. Protein binding assay by means of a surface plasmon resonance device

### (1) Experimental Method

The PD-L1 protein was attached to the surface of a gold sensor chip for surface plasmon resonance activated by EDC-NHS and blocked with ethanolamine (pH 8.5). PpNF and PpCF proteins dissolved in 20 mM Tris-HCl (pH 8.0), 150 mM NaCl, and 2 mM DTT solutions were each flowed over the gold sensor chip bound with PD-L1 proteins respectively to analyze binding kinetics. The right channel, without PD-L1 protein attached, was used as a reference to correct the resonance measurements.

### (2) Experimental Results

As a result of direct measurement of the binding of PpNF and PpCF with PD-L1, PpNF directly bound to PD-L1 (Kd = 39 nM), while PpCF showed no binding at all even at a concentration of 200 nM. Therefore, it was revealed that PpNF displayed PD-L1-binding peptide 1 in a structure capable of binding to PD-L1, which was used in future experiments (FIG. 3B).

### <Example 4> Observation of activity of PpNF after co-culture of cancer cells and immune cells to identify an immune checkpoint inhibitory function of PpNF in vitro

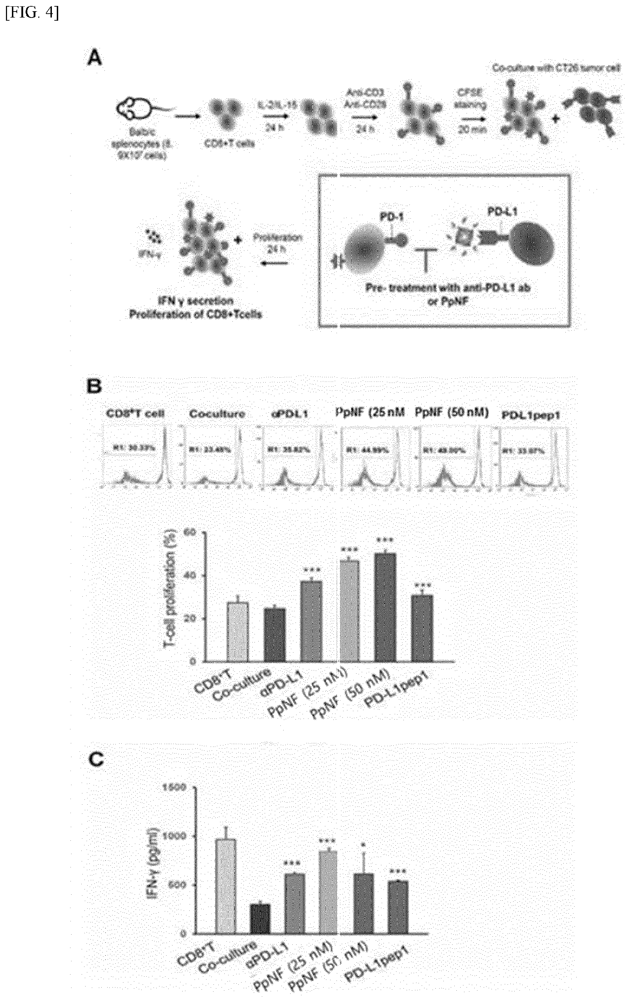

CD8⁺ T cells were extracted from the spleen obtained by sacrificing BALB/C mice. Extracted CD8⁺ T cells were treated with interleukin-2/interleukin-15 and anti-CD-3 antibody/anti-CD-28 antibody each for 24 hours for activation. Prior to treating activated CD8⁺ T cells with CFSE and performing symbiotic culture of tumor cells (CT26, mouse colon cancer), tumor cells were pretreated with anti-PD-L1 antibody, PpNF (25 nM or 50 nM), and PD-L1pep1 (50 nM), respectively, and interactions of PD-L1 of tumor cells and PD-L1 of activated CD8⁺ T cells were inhibited, respectively. After symbiotic culture for 24 hours, CD8⁺ T cells were isolated (FIG. 4A). The degree of CFSE binding to isolated T cells was measured with a flow cytometer (FIG. 4B). In addition, the amount of interferon gamma secreted by isolated T cells was measured using an enzyme-linked immunosorbent assay (FIG. 4C).

CD8⁺ T cells isolated from mice were activated (IL-2/IL-15 treatment and anti-CD3/anti-CD28 treatment), labeled with fluorescence (CFSE staining), and cultured with CT26 colorectal cancer cells. At this time, PD-L1 of cancer cells and PD-1 of T-cells were bound and showed an immunosuppressive effect. Treatment with PpNF or PD-L1 antibodies has shown immunoactive effects, i.e., T-cell proliferation and IFN-γ increment, by inhibiting PD-1/PD-L1 binding.

Thereby, it was possible to verify that PpNF had an immune checkpoint inhibitory effect, and it was found that the effect of PpNF is better than that of PD-L1 antibody or PD-L1 pep1 (PD-L1-binding peptide 1).

### <Example 5> Analysis of tumor targeting ability

1×10⁶ CT26 colorectal cancer cells contained in 100 µl of PBS were injected subcutaneously into the right thigh of BLAB/c mice aged 6 weeks. When the tumor was about 200 mm³ in size, 200 µl each of PpNF (10 mg/kg) that was labeled with Flamma-675 and dissolved in saline as well as ferritin wildtype (10 mg/kg) proteins was injected into each tail vein of the mice.

IVIS fluorescence imaging systems were used to investigate the in vivo distribution of proteins 1, 4, 8, 12, and 24 hours after injection. Then, after 24 hours, the mice were sacrificed and tumors, hearts, lungs, liver, kidneys, and spleen were taken out to investigate how many proteins remained in the organs.

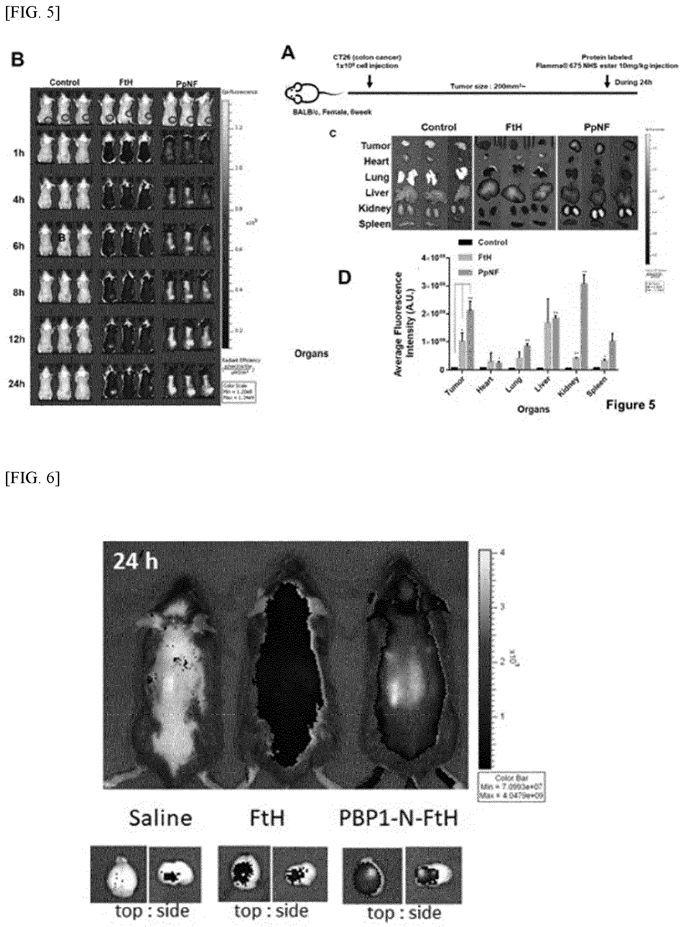

It was found that PpNF had increased tumor targeting ability than ferritin wildtype (tumor target by the EPR effect) (FIG. 5).

In addition, the glioblastoma targeting effect of the immune checkpoint blocking nanocage was identified. GL26, a glioblastoma cell line, was injected into the left side of the mouse brain to create an orthotopic GBM model and fluorescently labeled PBP1-N-FtH was injected into the tail vein [control: saline, ferritin]. After 24 hours, a fluorescence signal was observed in the brain, and it was observed that PBP1-N-FtH was targeted to the tumor site on the left side of the brain (FIG. 6).

### <Example 6> Analysis of anticancer and immune checkpoint inhibitory effects of PpNF in vivo

### 1. Experimental Method

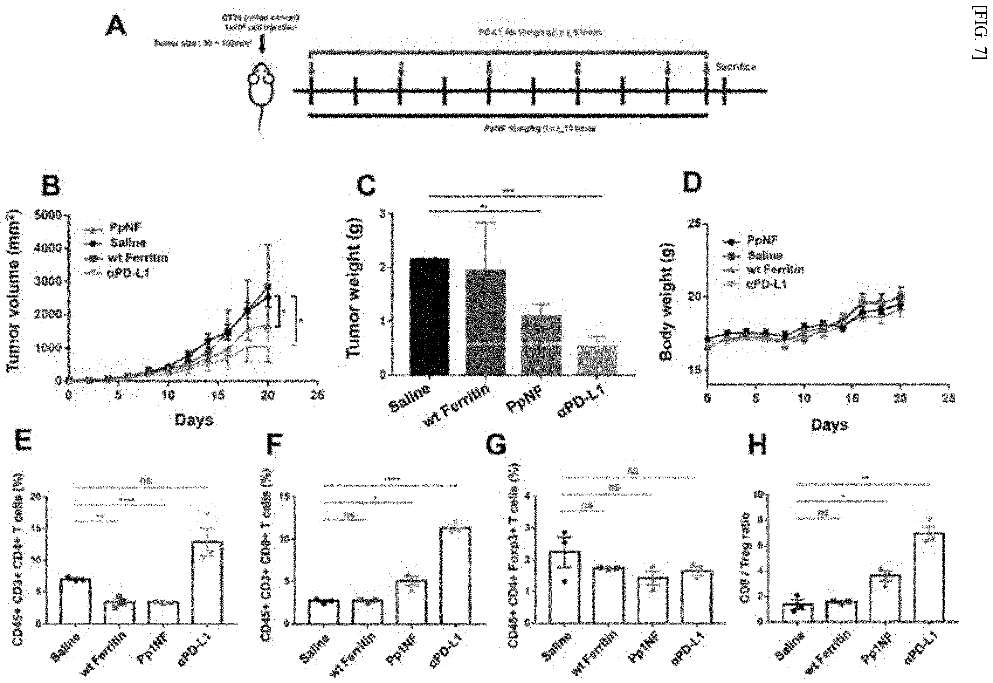

CT26 cells were injected subcutaneously into the upper right thigh of BALB/c wildtype mice. When the tumor was about 100 mm³ in size, mice were randomly divided into four groups. Saline, PpNF (10 mg/kg) dissolved in saline, and wildtype ferritin (10 mg/kg) were injected into the tail vein of each mouse, and therapeutic anti-PD-L1 antibody (10 mg/kg) was injected intraperitoneally (FIG. 7A). A total of 10 injections were performed once every two days (once every 4 days, repeated 6 times in total for anti-PD-L1 antibodies) to measure tumor size and weight of mice (FIGS. 7B and 7D). After 3 weeks, the tumor was removed by sacrificing mice and the tumor was weighed (FIG. 7C). The extracellular matrix of the removed tumor was degraded with collagenase to form single cells, and antibodies against labeled proteins of CD4⁺ T cells, CD8⁺ T cells, and regulatory T cells were each treated to analyze how many T cells are each present in the tumor by flow cytometry (FIGS. 7E to 7G).

### 2. Experimental Results

PpNF alone significantly inhibited tumor growth compared to the control (p<0.05) (FIG. 7B). The weight of the tumor was much smaller (FIG. 7C), and by increasing the proportion of CD8⁺ T cells in the tumor (FIG. 7F), the ratio of CD8⁺ T cells and regulatory T cells in the tumor was significantly increased compared to the control (FIG. 7H). Both PpNF and wildtype ferritin showed no change in body weight compared to control mice (FIG. 7D).

Through the results, it was found that PpNF had the effect of inhibiting the immune checkpoint in vivo, increasing CD8⁺ T cells, and eventually inhibiting tumor growth.

### <Example 7> Analysis of anticancer and immune checkpoint inhibitory effects in vivo in a combined therapy using PpNF and doxorubicin

### 1. Experimental Method

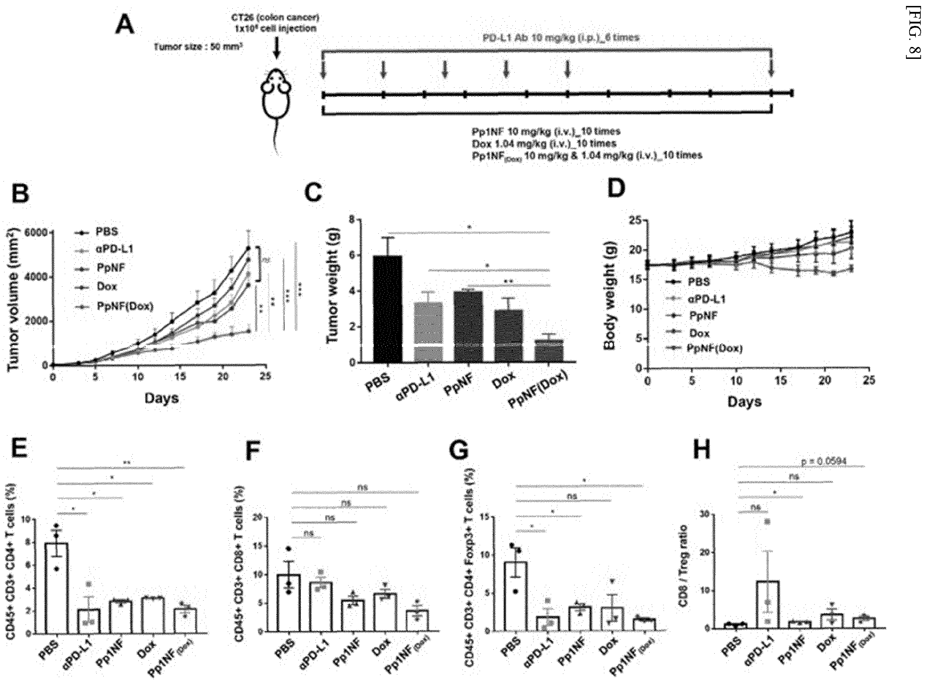

CT26 cells were injected subcutaneously into the upper right thigh of BALB/c wildtype mice. When the tumor was about 50 mm³ in size, they were randomly divided into five groups. PBS, PpNF (10 mg/kg) dissolved in PBS, PpNF (Dox) (10 mg/kg, 1.04 mg/kg for doxorubicin) in which doxorubicin was loaded in ferritin, and doxorubicin (1.04 mg/kg) were injected into the tail vein of each mouse, and therapeutic anti-PD-L1 antibody (10 mg/kg) was injected intraperitoneally (FIG. 8A). A total of 10 injections (once every 3 days, repeated 6 times in total for anti-PD-L1 antibodies,) were performed 3 times a week to measure tumor size and weight of mice (FIGS. 8B and 8D). After 3 weeks, the tumor was removed by sacrificing the mice, and the tumor was weighed (FIG. 8C). The extracellular matrix of the removed tumor was degraded with collagenase to form single cells, and antibodies against labeled proteins of CD4⁺ T cells, CD8⁺ T cells, and regulatory T cells were treated respectively to analyze how many T cells are each present in the tumor by flow cytometry (FIGS. 8E to 8G).

### 2. Experimental Results

When PpNF was loaded with doxorubicin and treated to the tumor in combined therapy, not only was the tumor growth significantly inhibited (p<0.001) compared to the control (p0.001), but also was the tumor growth significantly inhibited also compared to the same amount of PpNF (p<0.001), to the same amount of doxorubicin (p<0.01), and to therapeutic anti-PD-L1 antibodies (p<0.01) (FIG. 8B). The weight of tumor was also reduced (FIG. 8C), and by reducing the proportion of regulatory T cells in the tumor (FIG. 8G), the ratio of CD8⁺ T cells and regulatory T cells in the tumor increased compared to the control (FIG. 8H).

From the results, it was found that the combined therapy with doxorubicin loaded in PpNF had the effect of inhibiting the immune checkpoint in vivo, increasing the ratio of CD8⁺ T cells to regulatory T cells in the tumor, and eventually inhibiting the tumor growth.

### <Example 8> Determination of biotoxicity of PpNF via blood and serologic analysis

After antitumor animal experiments, blood and serum were collected from each group when sacrificing mice, and the amounts of red blood cells, hemoglobin, hematocrit, white blood cells, and neutrophils in the blood, blood urea nitrogen in serum, creatinine, alanine aminotransferase, and aspartate aminotransferase were measured and compared.

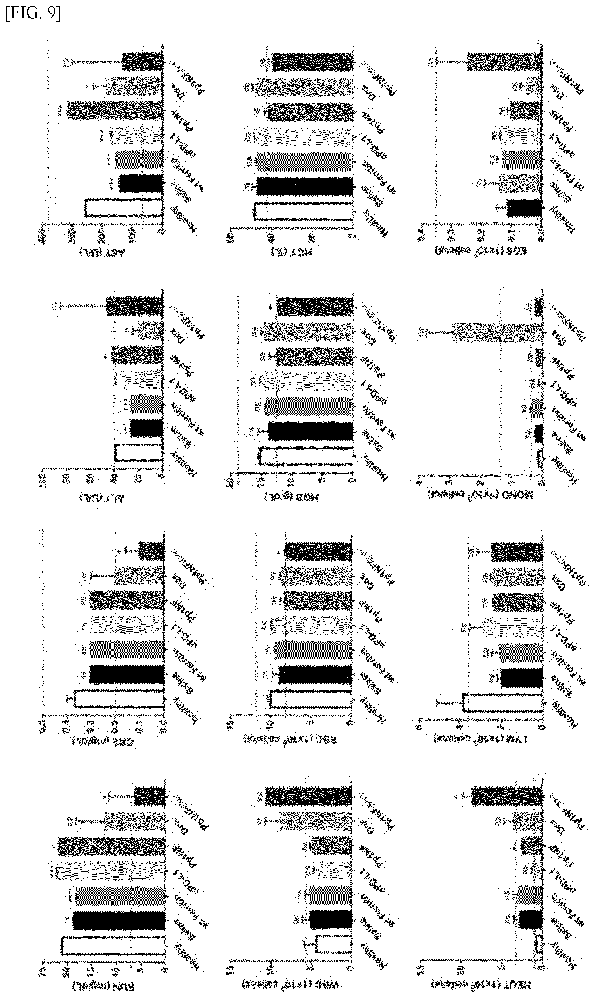

No hepatotoxicity or nephrotoxicity was observed in the mouse group treated with PpNF and PpNF with doxorubicin loaded, and there was no significant hematologic change other than an increase in neutrophils in PpNF loaded with doxorubicin (FIG. 9).

## Claims

1. A pharmaceutical composition for use in preventing or treating cancer, comprising a ferritin nanocage comprising a fusion polypeptide in which a PD-L1-binding peptide consisting of an amino acid sequence represented by SEQ ID NO: 2 is conjugated to a N-terminal of a human ferritin heavy chain fragment consisting of an amino acid sequence represented by SEQ ID NO: 1 as an active ingredient.

2. The pharmaceutical composition for use of claim 1, wherein the fusion polypeptide further comprises a linker between the human ferritin heavy chain fragment and the PD-L1-binding peptide.

3. The pharmaceutical composition for use of claim 2, wherein the linker has an amino acid sequence represented by SEQ ID NO: 3 or SEQ ID NO: 4.

4. The pharmaceutical composition for use of claim 1, wherein the ferritin nanocage is in a form in which the PD-L1-binding peptide is fused to an outer surface of ferritin.

5. The pharmaceutical composition for use of claim 1, wherein the cancer comprises any one selected from the group consisting of breast cancer, colon cancer, rectal cancer, brain tumor, lung cancer, liver cancer, skin cancer, esophageal cancer, testicular cancer, kidney cancer, stomach cancer, bladder cancer, ovarian cancer, cholangiocarcinoma, gallbladder cancer, uterine cancer, cervical cancer, prostate cancer, head and neck cancer, pancreatic cancer, and squamous cell carcinoma.

6. The pharmaceutical composition for use of claim 1, wherein the pharmaceutical composition blocks binding of PD-1 and PD-L1 and inhibits immune checkpoints.

7. The pharmaceutical composition for use of claim 1, further comprising an anticancer agent as active ingredient.

8. The pharmaceutical composition for use of claim 7, wherein the anticancer agent is any one or more selected from the group consisting of doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, and nitrosourea.

9. The pharmaceutical composition for use of claim 7, wherein the cancer is any one selected from the group consisting of breast cancer, colon cancer, rectal cancer, brain tumor, lung cancer, liver cancer, skin cancer, esophageal cancer, testicular cancer, kidney cancer, stomach cancer, bladder cancer, ovarian cancer, cholangiocarcinoma, gallbladder cancer, uterine cancer, cervical cancer, prostate cancer, head and neck cancer, pancreatic cancer, and squamous cell carcinoma.

## Patentansprüche

1. Pharmazeutische Zusammensetzung zur Verwendung bei der Vorbeugung oder Behandlung von Krebs, umfassend einen Ferritin-Nanokäfig, der ein Fusionspolypeptid umfasst, in dem ein PD-L1-bindendes Peptid, bestehend aus einer Aminosäuresequenz, die durch SEQ ID NO: 2 dargestellt ist, an ein N-Terminal eines menschlichen Ferritin-Schwerkettfragments konjugiert ist, das aus einer Aminosäuresequenz besteht, die durch SEQ ID NO: 1 dargestellt ist, als einen Wirkstoff.

2. Pharmazeutische Zusammensetzung zur Verwendung nach Anspruch 1, wobei das Fusionspolypeptid ferner einen Linker zwischen dem menschlichen Ferritin-Schwerkettfragment und dem PD-L1-bindenden Peptid umfasst.

3. Pharmazeutische Zusammensetzung zur Verwendung nach Anspruch 2, wobei der Linker eine Aminosäuresequenz besitzt, die durch SEQ ID NO: 3 oder SEQ ID NO: 4 dargestellt ist.

4. Pharmazeutische Zusammensetzung zur Verwendung nach Anspruch 1, wobei der Ferritin-Nanokäfig in einer Form vorliegt, in der das PD-L1-bindende Peptid an eine Außenfläche des Ferritins gebunden ist.

5. Pharmazeutische Zusammensetzung zur Verwendung nach Anspruch 1, wobei der Krebs einen umfasst, der aus der Gruppe ausgewählt ist, die aus Brustkrebs, Darmkrebs, Rektumkrebs, Hirntumor, Lungenkrebs, Leberkrebs, Hautkrebs, Speiseröhrenkrebs, Hodenkrebs, Nierenkrebs, Magenkrebs, Blasenkrebs, Eierstockkrebs, Gallengangskarzinom, Gallenblasenkrebs, Gebärmutterkrebs, Gebärmutterhalskrebs, Prostatakrebs, Kopf- und Halskrebs, Bauchspeicheldrüsenkrebs und Plattenepithelkarzinom besteht.

6. Pharmazeutische Zusammensetzung zur Verwendung nach Anspruch 1, wobei die pharmazeutische Zusammensetzung die Bindung von PD-1 und PD-L1 blockiert und Immun-Checkpoints hemmt.

7. Pharmazeutische Zusammensetzung zur Verwendung nach Anspruch 1, ferner umfassend ein Antikrebsmittel als Wirkstoff.

8. Pharmazeutische Zusammensetzung zur Verwendung nach Anspruch 7, wobei das Antikrebsmittel eines oder mehrere sind, die aus der Gruppe ausgewählt sind, die aus Doxorubicin, Paclitaxel, Vincristin, Daunorubicin, Vinblastin, Actinomycin-D, Docetaxel, Etoposid, Teniposid, Bisantren, Homoharringtonin, Gleevec (STI-571), Cisplatin, 5-Fluorouracil, Adriamycin, Methotrexat, Busulfan, Chlorambucil, Cyclophosphamid, Melphalan, Stickstofflost und Nitrosoharnstoff besteht.

9. Pharmazeutische Zusammensetzung zur Verwendung nach Anspruch 7, wobei der Krebs einer ist, der aus der Gruppe ausgewählt ist, die aus Brustkrebs, Darmkrebs, Rektumkrebs, Hirntumor, Lungenkrebs, Leberkrebs, Hautkrebs, Speiseröhrenkrebs, Hodenkrebs, Nierenkrebs, Magenkrebs, Blasenkrebs, Eierstockkrebs, Gallengangskarzinom, Gallenblasenkrebs, Gebärmutterkrebs, Gebärmutterhalskrebs, Prostatakrebs, Kopf- und Halskrebs, Bauchspeicheldrüsenkrebs und Plattenepithelkarzinom besteht.

## Revendications

1. Composition pharmaceutique pour utilisation dans la prévention ou le traitement du cancer, comprenant une nanocage de ferritine comprenant un polypeptide de fusion dans lequel un peptide se liant au PD-L1 constitué d'une séquence d'acides aminés représentée par SEQ ID NO : 2 est conjugué à une extrémité N-terminale d'un fragment de chaîne lourde de ferritine humaine constitué d'une séquence d'acides aminés représentée par SEQ ID NO : 1 en tant qu'ingrédient actif.

2. Composition pharmaceutique pour utilisation selon la revendication 1, dans laquelle le polypeptide de fusion comprend en outre un lieur entre le fragment de chaîne lourde de ferritine humaine et le peptide se liant au PD-L1.

3. Composition pharmaceutique pour utilisation selon la revendication 2, dans laquelle le lieur a une séquence d'acides aminés représentée par SEQ ID NO : 3 ou SEQ ID NO : 4.

4. Composition pharmaceutique pour utilisation selon la revendication 1, dans laquelle la nanocage de ferritine est dans une forme dans laquelle le peptide se liant au PD-L1 est fusionné à une surface externe de la ferritine.

5. Composition pharmaceutique pour utilisation selon la revendication 1, dans laquelle le cancer comprend l'un quelconque choisi parmi le groupe consistant en cancer du sein, cancer du côlon, cancer rectal, tumeur cérébrale, cancer du poumon, cancer du foie, cancer de la peau, cancer de l'œsophage, cancer des testicules, cancer du rein, cancer de l'estomac, cancer de la vessie, cancer de l'ovaire, cholangiocarcinome, cancer de la vésicule biliaire, cancer de l'utérus, cancer du col de l'utérus, cancer de la prostate, cancer de la tête et du cou, cancer du pancréas et carcinome épidermoïde.

6. Composition pharmaceutique pour utilisation selon la revendication 1, dans laquelle la composition pharmaceutique bloque la liaison de PD-1 et PD-L1 et inhibe les points de contrôle immunitaires.

7. Composition pharmaceutique selon la revendication 1, comprenant en outre un agent anticancéreux en tant qu'ingrédient actif.

8. Composition pharmaceutique pour utilisation selon la revendication 7, dans laquelle l'agent anticancéreux est un ou plusieurs agents choisis parmi le groupe consistant en doxorubicine, paclitaxel, vincristine, daunorubicine, vinblastine, actinomycine-D, docétaxel, étoposide, téniposide, bisantrène, homoharringtonine, Gleevec (STI-571), cisplatine, 5-fluorouracile, adriamycine, méthotrexate, busulfan, chlorambucil, cyclophosphamide, melphalan, moutarde azotée et nitrosourée.

9. Composition pharmaceutique selon la revendication 7, dans laquelle le cancer est choisi parmi le groupe consistant en cancer du sein, cancer du côlon, cancer du rectum, tumeur cérébrale, cancer du poumon, cancer du foie, cancer de la peau, cancer de l'œsophage, cancer des testicules, cancer du rein, cancer de l'estomac, cancer de la vessie, cancer de l'ovaire, cholangiocarcinome, cancer de la vésicule biliaire, cancer de l'utérus, cancer du col de l'utérus, cancer de la prostate, cancer de la tête et du cou, cancer du pancréas et carcinome épidermoïde.
